# EUROPEAN PATENT SPECIFICATION

(11) **EP 1 941 927 B1**
(45) Date of publication and mention of the grant of the patent: **30.08.2017**
(21) Application number: 08152518.0
(22) Date of filing: 28.06.2002
(51) Int. Cl.: A61M 25/00

(54) **A method of producing a catheter and a catheter**
Katheter und Methode zum Herstellen eines Katheters
Procédé de fabrication de cathéter et cathéter

(30) Priority: 29.06.2001 DK 200101041; 29.06.2001 US 893514; 24.09.2001 DK 200101386; 13.12.2001 DK 200101869; 13.12.2001 DK 200101870; 27.12.2001 US 26819; 17.04.2002 DK 200200569; 17.04.2002 DK 200200570; 13.06.2002 DK 200200895
(43) Date of publication of application: 09.07.2008
(62) Divisional of application: 02754547.4
(73) Proprietor: Coloplast A/S, 3050 Humlebæk (DK)
(72) Inventor: Tanghøj, Allan, DK-2980, Kokkedal (DK); Jensen, Lars Bøgelund, DK-2610, Rødovre (DK)

(56) References cited:
- EP-A- 0 925 802
- EP-A2- 1 116 567
- WO-A-90/00960
- DE-C- 4 140 099
- US-A- 3 865 666
- US-A- 4 041 122

## Description

### Field of the invention

The present invention relates to a method of producing a medical catheter. In particular, the invention relates to a method wherein a catheter is produced by solidifying a fluid catheter material in a mould.

### Background of the invention

In general, medical catheters are used for draining bodily fluids such as blood and urine. A catheter for medical use is typically provided with a tubular oblong catheter body part made from a piece of flexible medical hose with an internal conduit, e.g. a PVC or PU hose having a substantially circular outer and inner cross-sectional shape. In an insertable end thereof, the catheter forms one or more openings through which fluid can drain from a bodily cavity and into the tubular body. In order to ease the insertion and to avoid injuring the bodily tissue when the catheter is inserted into a body opening and guided through a bodily canal, e.g. the urethra or a blood vessel, the insertable end is normally provided with a smoothly rounded tip. In the case of most catheters, the tip is formed by heating and melting an end part of the medical hose until its conduit seals. Even though the existing catheters are formed with a tip providing an acceptably safe and comfortable insertion, it is a desire to further shape a larger and more curved tip of the inserted part of the catheter since this may allow for an easier and safer insertion. However, the present technique of forming the tip of a medial hose does not support in making such a tip. As an alternative, some catheters are made from a medical hose with a glued-on catheter tip.
This solution allows for a catheter with a larger and more curved tip, but the additional process step of providing a tip part and gluing the part to the medical hose implies additional production costs. Moreover, there is potential risk that the tip falls off during the catheterisation and thus remains inside the body cavity, e.g. in the bladder. For economical and for safety reasons, the on-glued catheter tip is therefore undesired.

When the tip has been formed, a number of draining holes are normally drilled or punched radially into the hose in the vicinity of the tip. In order to avoid that the holes damage the bodily tissue during the insertion of the catheter, the edges of the holes must be smoothly rounded. Accordingly, the process of making the holes is time-consuming and expensive.

At its opposite end, the oblong catheter body is formed with an opening allowing the fluid to drain out of the catheter and into an appropriate place of disposal. In this opposite end, most catheters are provided with a connector part. The connector part allows the catheter to be connected e.g. to a bag for collecting the fluid. The connector part is normally formed by adhesively bonding a funnel-shaped member to the medical hose. Once again, the additional process of gluing a separate member onto the hose is cost-inefficient and implies a larger percentage of defect products.

A prior art method is disclosed in EP1116567.

### Description of the invention

It is an object of the present invention to overcome the above described problems by providing a method of producing a catheter as disclosed in claim 1, said method comprising the steps of:
- injecting a fluid catheter material into a mould formed to define an insertable catheter tip and a catheter body in one part, and subsequently
- solidifying the material therein.

The injection may take place in a regular machine for injection moulding. Depending upon the size and length of the catheter, the injection pressure may be in the range of 500-1500 bar, such as 750-1250 bar such as in the size of 1000 bar.

The injection moulding process is in particular suitable for relatively short catheters, i.e. catheters which are in the range of 50-90 mm., such as in the range of 55-85 mm., such as in the range of 60-80 mm. such as with a length in the size of 70 mm. which length has been found to be a suitable insertable length for most female individuals. For male individuals, catheter sections may preferably be provided in a length in the range of 180-250 mm., such as in the range of 190-240 mm., such as in the range of 200-230 mm. such as in the size of 220 mm.

The catheter may further comprise connection means for connecting the proximal insertion section to a further catheter section or to a urinary collection bag. The connector part may be made from the same material as the proximal insertion section, whereby, at the step of forming the proximal insertion section, the proximal insertion section and the connector part may be formed substantially simultaneously. Alternatively, the connector part may be made from a material different from the material of the proximal insertion section, whereby the connector part and the proximal insertion section are formed in distinct process steps, for example in a multi-component injection moulding process.

The mould could be formed to define the body part as an oblong hollow, tubular part with an internal conduit of a size allowing bodily fluid to be drained through the catheter body. As an alternative, the body part could be provided in the form of an oblong solid kernel with one or more vanes extending radially from the kernel and along the entire length thereof. The vanes thus define a number of draining passages for draining urine between the kernel and a bodily draining passage, e.g. the urethra.
In order to allow the bodily fluid, e.g. urine from the bladder, to enter the hollow, tubular body part of the catheter, the mould could be formed to define at least one draining hole in the vicinity of the tip.

A connector part may be provided for connecting the catheter to a hose for extending the length of the catheter or for connecting the catheter to disposal means, e.g. to a urinary collection bag. The connector section could preferably be made in one part with the catheter body and the tip. The connector part may be made from the same material as the proximal insertion section and preferably substantially simultaneous therewith, e.g. during the same injection step. Alternatively, the connector part may be made from a material different from the material of the proximal insertion section. The connector part and the proximal insertion section are thus formed in distinct process steps, for example in a multicomponent injection moulding process. The connector part may also be arranged as a separate component in the injection mould before the injection of the catheter material so that the connector part is moulded into engagement with the catheter body part during the injection moulding of the body part of the catheter. In a similar manner, a catheter tip may be arranged as a separate component in the mould and, during the injection moulding of the body part of the catheter, be moulded into the catheter. In a similar manner, additional components may be arranged in the mould prior to the injection moulding of the catheter body. As an example, one or more ring-shaped coloured members may be arranged for the purpose of visualising a certain length of the catheter, e.g. for visualising the intended insertable length. As another example, one or more objects made of a material, which improves the visualisation of the catheter in an x-ray or ultra-sound image may be arranged in the mould prior to the injection moulding of the catheter body.

In order to allow a user of the catheter to get a better grip, the catheter may be provided with means for attaching the catheter to peripheral articles such as a hand-grip for firmly gripping the catheter. The means for attaching the catheter to peripheral articles could be an out or inwardly extending bulge. As a further option, the catheter could be formed in one piece with means for handling the catheter during the insertion, e.g. a handle part which supports for a firm hand grip. As an example, the catheter could be provided in a diameter allowing for insertion into the urethra and with a catheter section not adapted for insertion and provided in a much larger diameter allowing a firm hand grip in the catheter.

Depending upon the type of catheterisation, the bodily fluid is typically drained either into a place of disposal e.g. into a toilet or into a reservoir or container for collecting the fluid. Accordingly, the mould could be formed to further define a reservoir for collecting the bodily fluids. The reservoir could be a plastic bag moulded in one piece with the catheter, e.g. by a combined injection and blow moulding process.

In order to fixate the catheter in the bodily passage, the catheter could be provided with a balloon in the vicinity of the inserted tip. The balloon could be moulded into one piece with the catheter.

Sometimes, it is desired that different parts of the catheter is provided with different characteristics. As an example, it may be desired for a urinary catheter that the insertable part is relatively soft and flexible so that the catheter can pass through the curved passage of the urethra. On the other hand, those parts of the catheter which is not adapted for insertion into the urethra may preferably be relatively less flexible, thus allowing an easier grip and allowing the inserted part of the catheter to be manipulated via the not inserted part.

Similarly, it may often be desired that the outer surface of the catheter is provided in a low-frictional material supporting an easier and more comfortable insertion of the catheter into the urinary canal. Accordingly, it may be desired to provide a surface layer of the catheter in a low-friction material such as FEP, PTFE or in a hydrophilic material such as polyvinylidone while the remaining part of the catheter is provided in a stronger and more durable material such as a thermoplastic elastomeric material, other thermoplastic materials, curable elastomeric materials, polyamide resins or elastomers or any mixture thereof, i.e. the group may comprise materials like, PVC, PU, PE, latex, and/or Kraton ™. As an example, the mould may be coated with a hydrophilic material prior to the injection of a thermoplastic elastomeric material into the mould. As an alternative, series of injections of one or more types of thermoplastic elastomeric materials into the mould may take place. As an example, a hydrophilic material or a similar low frictional material such as siloxane, FEP etc. may firstly be injected to form an outer layer of the catheter. Subsequently, one or more types of materials, e.g. materials with different characteristics, are injected in one or more injection cycles in order to form the rest of the catheter.

As an advantage of the injection moulding process, the surface of the catheter may easily be provided with a surface roughness defining anchoring points for adhering a low frictional material to the surface of the catheter, e.g. anchoring points for bonding a FEP or PTFE (Teflon™) coating to the surface.

When injecting the material in more than one injection cycle, a catheter comprising multiple layers or laminates and which additionally may comprise more individual sections in the length, is formed. Due to the laminated structure, the laminated structure may be more durable towards mechanical stress. Due to the individual sections in the length, a first part of the catheter, e.g. the part of the catheter which is intended to be inserted into the urethra or similar body canal, may be made in a softer material than the remaining catheter or it may be made in another colour or with another slipperiness, i.e. more slippery than the parts not intended to be inserted.

The different characteristics may relate to the softness of the material after solidification, the colour of the material, the strength of the material, the slipperiness of the material after solidification or to the ability of the solidified material to absorb liquid substances.

As an example, the part of the catheter which is adapted for insertion into the body canal, may be coloured in a colour different from the colour of the other parts of the catheter. In that way, the user can easily avoid touching the insertable part of the catheter.

In order to reduce the resistance against insertion into the body canal, the radial size of the tip may be larger than the radial size of the rest of the catheter. As an example, the tip may be formed into an onion-shaped or bulbous knob or the tip may be formed with a conical shape. Preferably, the tip is provided with a first end part, fronting the body canal opening during the insertion and provided with a narrow radial size. From this end part, the tip widens out in an intermediate part until the radial size exceeds the radial size of the catheter body part. Between the intermediate part of the tip and the catheter body part, the radial size slopes down in a second end part until the radial size of the catheter body part.

Inlet openings for draining bodily fluid from a body cavity and into the catheter may be provided either in the first end part, in the intermediate part or in the second end part or in more than one part or even en all of the parts. As an example, the tip may be formed in with a plurality of small holes in the tip.

According to a second aspect, the present invention relates to a catheter formed by the above described method and comprising the above described features.

### Brief description of the drawings

Preferred embodiments of the invention will now be described in details with reference to the drawing in which:
Fig. 1 shows an injection-moulded catheter with a tip and a connector,
Fig. 2 shows a bulbous catheter tip,
Fig. 3 shows a conical catheter tip,
Fig. 4 shows a spherical catheter tip,
Fig. 5 shows a catheter with a reservoir for collecting bodily fluids, and
Fig. 6 shows a catheter in the form of an oblong solid kernel with a plurality of vanes extending radially from the kernel and along the entire length thereof.

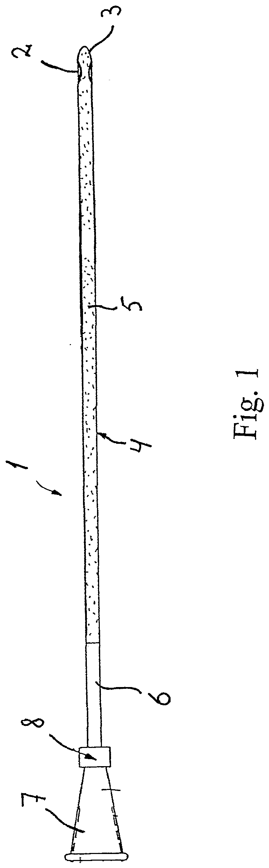

Referring to Fig. 1, the invention relates to a method of producing a catheter 1 by injecting a fluid catheter material into a mould formed to define an insertable catheter tip 3 and a catheter body 4 and subsequently solidifying the material therein. As shown, a catheter connector section 7 may be formed in one part with the catheter body. Furthermore, means 8 for attaching the catheter to peripheral articles for easing the handling of the catheter could be formed in one part with the catheter body during the moulding process.

In the insertable end, i.e. in the vicinity of the tip, a draining hole 2 allows bodily fluids to flow from a body cavity and into an internal conduit of the catheter. The internal conduit transports the fluid to the connector part where the fluid can be. disposed into a bag or into a place of disposal.

The catheter of Fig. 1, is on its insertable part provided with a friction reducing material 5 and on its non-insertable part with a highly frictional material 6. The friction reducing material supports in a safe and easy insertion of the catheter into a body canal such as the urethra and the highly frictional material supports an easier grip in the non-inserted part and thus supports for easier manipulation of the catheter. The characteristics of the materials 5 and 6, respectively, could also relate to different colours indicating to the user which part of the catheter is intended to be inserted. The characteristics could also relate to different resiliency of the insertable part versus the non-insertable part e.g. allowing the inserted part easily to follow a curved body canal, e.g. for passing through the urethra around the prostate.

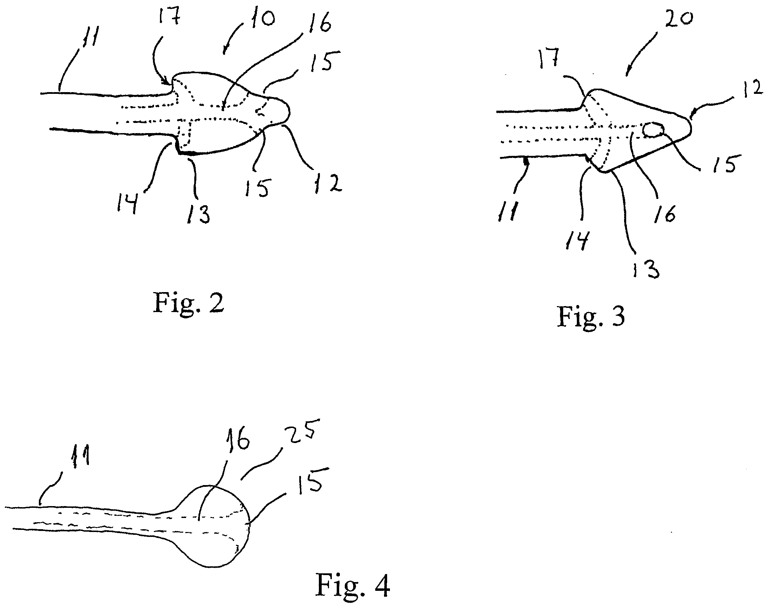

Figs. 2, 3 and 4 shows three different alternative tip shapes, i.e. a bulbous tip 10 of Fig. 2, a conical tip 20 of Fig. 3 and a spherical tip 25 of Fig. 4. The tip is provided in the inserted 35 end of the catheter body 11 in one part with the body and during the moulding process. The tip is formed with a first end part 12 with a radial size which is smaller than the radial size of the catheter body, an intermediate part 13 with a radial size which is larger than the radial size of the catheter and a second end part 14 wherein the size slopes down to the radial size of the catheter. In order to allow bodily fluids to drain into an internal conduit of the catheter, draining holes are provided in one or more of the parts of the tip. In Figs. 2 and 3, draining holes 15 are provided in the first end part and draining holes 17 are provided in the second end part. The tip is internally moulded with a conduit 16 for guiding the fluid to the body part of the catheter. As shown in Fig. 4, a draining hole or holes may be provided in the tip, so that the opening point in the axial direction of the catheter body. In that case it is important that edges of the hole or holes are smoothly rounded in order not to injure the body canal.

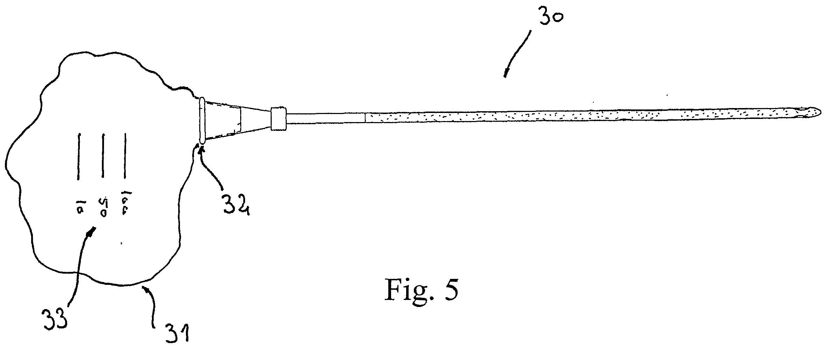

Fig. 5 shows a catheter 30 and a reservoir 31 formed by moulding in one part for collecting the bodily fluids, e.g. blood or urine. The reservoir could be formed during the injection moulding process by combining the injection moulding process with blow moulding. During this process, pressurised gas is used for expanding the reservoir part of the catheter product into a plastic bag or plastic bottle shaped item. The reservoir may, during the moulding process, be provided with filling level indication marks 33 and may internally be provided with a hydrophilic material for the conversion of a liquid substance into a substantially solid or gel-like substance.

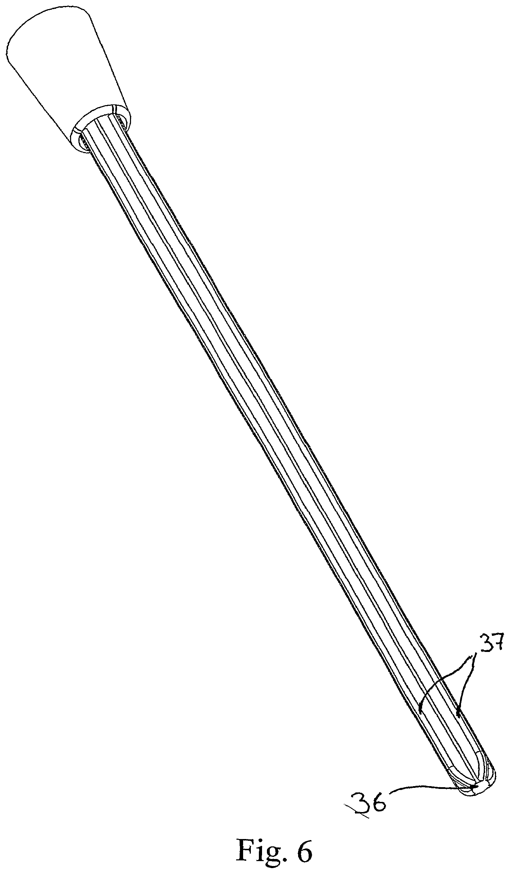

Fig. 6 shows catheter in the form of a solid kernel 36 with a plurality of radially extending vanes 37. The vanes form a number of draining passages for draining urine between the kernel and a bodily draining passage, e.g. the urethra.

## Claims

1. A method for producing a urinary catheter (1) with parts having different characteristics, said method comprising the steps of:
- injecting at least two different fluid catheter materials into a mould formed to define an insertable catheter tip (3) and body (4) and subsequently
- solidifying the material therein to form a catheter having at least two layers.

2. A method according to claim 1, wherein the at least two layers form a catheter having a laminated structure.

3. A method according to any of the preceding claims, wherein the at least two layers are multiple layers.

4. A method according to any of the preceding claims, wherein the catheter material is injected into the mould in more than one injection cycle.

5. A method according to any of the preceding claims, wherein the difference in the characteristics relates to the softness of the materials after solidification.

6. A method according to any of the preceding claims, wherein the difference in the characteristics relates to the colours of the material.

7. A method according to any of the preceding claims, wherein the difference in the characteristics relates to the strengths of the materials.

8. A method according to any of the preceding claims, wherein the difference in the characteristics relates to the slipperiness of the materials after solidification.

9. A method according to any of the preceding claims, wherein the difference in the characteristics relates to the ability of the solidified materials to absorb liquid substances.

10. A method according to any of the preceding claims, wherein the body (4) has an internal conduit and the insertable tip (3) seals off the end part of the conduit.

11. A method according to any of the preceding claims, wherein the mould is formed to define at least one draining hole in the vicinity of the tip.

12. A method according to any of the preceding claims, wherein the catheter is provided with a connector part made during the same injection moulding step as the catheter.

13. A method according to any of the preceding claims, wherein a connector part is arranged as a separate component in the injection mould before the injection of the catheter material so that the connector part is moulded into engagement with the catheter body part during the injection moulding of the body part of the catheter.

14. An injection moulded urinary catheter (1) with an insertable catheter tip (3) and body (4), the catheter being made of at least two different catheter materials, the materials being joined during the moulding process in which both materials are injected into a mould in fluid state and solidified therein to form a catheter having at least two layers.

15. A catheter according to claim 14, wherein the at least two layers form a catheter having a laminated structure.

16. A catheter according to claims 14 or 15, wherein the at least two layers are multiple layers.

17. A catheter according to any of the claims 14-16, wherein the body (4) has an internal conduit and the insertable tip (3) seals off the end part of the conduit.

18. A catheter according to any of the claims 14-17, wherein the catheter body has a first radial size, and wherein the tip has an intermediate part (13) with a second radial size, the second radial size being larger than the first radial size.

19. A catheter according to claim 18, comprising at least one opening (17) between the intermediate part of the tip and the body for draining body fluid from a body cavity into the catheter.

20. A catheter according to claim 18 or 19, wherein the tip has a narrow part (12) with a third radial size which is smaller than the second radial size, the catheter comprising at least one opening (15) between the narrow part and the intermediate part for draining body fluid from a body cavity into the catheter.

21. A catheter according to any of claims 14-20, wherein at least one of the materials is a hydrophilic polymer material.

22. A catheter according to any of claims 14-21, comprising at least one draining hole in the vicinity of the tip.

23. A catheter according to any of claims 14-22, comprising means for handling the catheter during insertion.

24. A catheter according to any of claims 14-23 further comprising a connector part.

25. A urinary catheter according to any of claims 14-24, produced according to any of the claims 1-13.

## Patentansprüche

1. Verfahren zur Herstellung eines Harnkatheters (1) mit Teilen mit unterschiedlichen Eigenschaften, das folgende Schritte umfasst:
- Einspritzen von mindestens zwei verschiedenen flüssigen Kathetermaterialien in eine Form, die so ausgebildet ist, dass sie eine einführbare Katheterspitze (3) und einen Körper (4) vorgibt, und anschließend
- Verfestigen des Materials darin, um einen Katheter mit mindestens zwei Schichten zu bilden.

2. Verfahren nach Anspruch 1, wobei die mindestens zwei Schichten einen Katheter mit einer laminierten Struktur bilden.

3. Verfahren nach einem der vorhergehenden Ansprüche, wobei die mindestens zwei Schichten Mehrfachschichten sind.

4. Verfahren nach einem der vorhergehenden Ansprüche, bei dem das Kathetermaterial in mehr als einem Einspritzzyklus in die Form eingespritzt wird.

5. Verfahren nach einem der vorhergehenden Ansprüche, bei dem sich der Unterschied in den Eigenschaften auf die Weichheit der Materialien nach der Verfestigung bezieht.

6. Verfahren nach einem der vorhergehenden Ansprüche, bei dem sich der Unterschied in den Eigenschaften auf die Farben des Materials bezieht.

7. Verfahren nach einem der vorhergehenden Ansprüche, bei dem sich der Unterschied in den Eigenschaften auf die Festigkeiten der Materialien bezieht.

8. Verfahren nach einem der vorhergehenden Ansprüche, bei dem sich der Unterschied in den Eigenschaften auf die Gleitfähigkeit der Materialien nach der Verfestigung bezieht.

9. Verfahren nach einem der vorhergehenden Ansprüche, bei dem sich der Unterschied in den Eigenschaften auf die Fähigkeit der verfestigten Materialien bezieht, flüssige Substanzen zu absorbieren.

10. Verfahren nach einem der vorhergehenden Ansprüche, wobei der Körper (4) eine innere Leitung hat und die einführbare Spitze (3) den Endteil der Leitung abdichtet.

11. Verfahren nach einem der vorhergehenden Ansprüche, wobei die Form so ausgebildet wird, dass sie mindestens ein Ablaufloch in der Nähe der Spitze definiert.

12. Verfahren nach einem der vorhergehenden Ansprüche, wobei der Katheter mit einem Verbinderteil versehen wird, der während desselben Spritzgießschritts wie der Katheter hergestellt worden ist.

13. Verfahren nach einem der vorhergehenden Ansprüche, wobei vor dem Einspritzen des Kathetermaterials ein Verbinderteil als eine separate Komponente in der Spritzgießform angeordnet wird, so dass der Verbinderteil während des Spritzgießens des Körperteils des Katheters so geformt wird, dass er mit dem Katheterkörperteil in Eingriff steht.

14. Durch Spritzgießen geformter Harnkatheter (1) mit einer einführbaren Katheterspitze (3) und einem Körper (4), wobei der Katheter aus mindestens zwei unterschiedlichen Kathetermaterialien hergestellt ist und die Materialien während des Formgebungsprozesses miteinander verbunden werden, bei dem die beiden Materialien in flüssigem Zustand in eine Form eingespritzt werden und sich darin verfestigen, um einen Katheter mit mindestens zwei Schichten zu bilden.

15. Katheter nach Anspruch 14, wobei die mindestens zwei Schichten einen Katheter mit einer laminierten Struktur bilden.

16. Katheter nach Ansprüchen 14 oder 15, wobei die mindestens zwei Schichten Mehrfachschichten sind.

17. Katheter nach einem der Ansprüche 14 - 16, wobei der Körper (4) eine innere Leitung hat und die einführbare Spitze (3) den Endteil der Leitung abdichtet.

18. Katheter nach einem der Ansprüche 14 bis 17, bei dem der Katheterkörper eine erste radiale Größe aufweist und bei dem die Spitze einen Zwischenteil (13) mit einer zweiten radialen Größe aufweist, wobei die zweite radiale Größe größer ist als die erste radiale Größe.

19. Katheter nach Anspruch 18, der mindestens eine Öffnung (17) zwischen dem Zwischenteil der Spitze und dem Körper zur Drainage von Körperflüssigkeit aus einem Körperhohlraum in den Katheter hinein aufweist.

20. Katheter nach Anspruch 18 oder 19, bei dem die Spitze einen schmalen Teil (12) mit einer dritten radialen Größe aufweist, die kleiner ist als die zweite radiale Größe, und der Katheter mindestens eine Öffnung (15) zwischen dem schmalen Teil und dem Zwischenteil zur Drainage von Körperflüssigkeit aus einem Körperhohlraum in den Katheter hinein aufweist.

21. Katheter nach einem der Ansprüche 14 - 20, bei dem mindestens eines der Materialien ein hydrophiles Polymermaterial ist.

22. Katheter nach einem der Ansprüche 14 - 21, umfassend mindestens ein Ablaufloch in der Nähe der Spitze.

23. Katheter nach einem der Ansprüche 14 - 22, umfassend Mittel zur Handhabung des Katheters während des Einführens.

24. Katheter nach einem der Ansprüche 14 - 23, ferner umfassend einen Verbinderteil.

25. Harnkatheter nach einem der Ansprüche 14 - 24, der nach einem der Ansprüche 1 - 13 hergestellt worden ist.

## Revendications

1. Procédé de fabrication d'un cathéter urinaire (1) qui est constitué de pièces présentant des caractéristiques différentes, ledit procédé comprenant les étapes suivantes :
- injecter, à l'état fluide, au moins deux matériaux différents entrant dans la composition d'un cathéter dans un moule agencé de manière à définir un embout de cathéter insérable (3) et une partie formant corps (4) et ensuite
- laisser les matériaux se solidifier dans celui-ci de façon à former un cathéter comportant au moins deux couches.

2. Procédé selon la revendication 1, dans lequel les au moins deux couches forment un cathéter présentant une structure stratifiée.

3. Procédé selon l'une quelconque des revendications précédentes, dans lequel les au moins deux couches sont de multiples couches.

4. Procédé selon l'une quelconque des revendications précédentes, dans lequel le matériau qui entre dans la composition du cathéter est injecté dans le moule en plus d'un cycle d'injection.

5. Procédé selon l'une quelconque des revendications précédentes, dans lequel la différence de caractéristiques réside dans la douceur des matériaux après leur solidification.

6. Procédé selon l'une quelconque des revendications précédentes, dans lequel la différence de caractéristiques réside dans les couleurs des matériaux.

7. Procédé selon l'une quelconque des revendications précédentes, dans lequel la différence de caractéristiques réside dans les propriétés de résistance mécanique des matériaux.

8. Procédé selon l'une quelconque des revendications précédentes, dans lequel la différence de caractéristiques réside dans le pouvoir glissant des matériaux après leur solidification.

9. Procédé selon l'une quelconque des revendications précédentes, dans lequel la différence de caractéristiques réside dans la capacité des matériaux solidifiés à absorber des substances liquides.

10. Procédé selon l'une quelconque des revendications précédentes, dans lequel le corps (4) comporte un conduit intérieur et l'embout insérable (3) obture la partie d'extrémité du conduit.

11. Procédé selon l'une quelconque des revendications précédentes, dans lequel le moule est formé de façon à définir au moins un orifice de drainage à proximité de l'embout.

12. Procédé selon l'une quelconque des revendications précédentes, dans lequel le cathéter est pourvu d'une pièce de raccordement réalisée au cours de la même étape de moulage par injection que le cathéter.

13. Procédé selon l'une quelconque des revendications précédentes, dans lequel une pièce de raccordement est placée sous forme de composant distinct dans le moule d'injection avant l'injection du matériau de cathéter de telle sorte que la pièce de raccordement soit moulée de façon à se solidariser avec la partie formant corps du cathéter au cours du moulage par injection de la partie formant corps du cathéter.

14. Cathéter urinaire moulé par injection (1), comprenant un embout de cathéter insérable (3) et une partie formant corps (4), ce cathéter étant constitué d'au moins deux matériaux différents entrant dans la composition d'un cathéter, les matériaux étant assemblés lors du processus de moulage dans lequel les deux matériaux sont injectés dans un moule à l'état liquide et se solidifient dans celui-ci de façon à former un cathéter comportant au moins deux couches.

15. Cathéter selon la revendication 14, dans lequel les au moins deux couches forment un cathéter présentant une structure stratifiée.

16. Cathéter selon les revendications 14 ou 15, dans lequel les au moins deux couches sont de multiples couches.

17. Cathéter selon l'une quelconque des revendications 14 à 16, dans lequel le corps (4) comporte un conduit intérieur et l'embout insérable (3) obture la partie d'extrémité du conduit.

18. Cathéter selon l'une quelconque des revendications 14 à 15, dans lequel le corps du cathéter présente une première dimension radiale et dans lequel l'embout comporte une portion intermédiaire (13) présentant une deuxième dimension radiale, la deuxième dimension radiale étant en l'occurrence plus grande que la première dimension radiale.

19. Cathéter selon la revendication 18, comprenant au moins une ouverture (17) entre la portion intermédiaire de l'embout et le corps pour permettre à un fluide corporel provenant d'une cavité corporelle de s'écouler dans le cathéter.

20. Cathéter selon la revendication 18 ou 19, dans lequel l'embout comporte une portion étroite (12) qui présente une troisième dimension radiale qui est plus petite que la deuxième dimension radiale, ce cathéter comportant au moins une ouverture (15) située entre la portion étroite et la portion intermédiaire pour permettre à un fluide corporel provenant d'une cavité corporelle de s'écouler dans le cathéter.

21. Cathéter selon l'une quelconque des revendications 14 à 20, dans lequel au moins un des matériaux est un polymère hydrophile.

22. Cathéter selon l'une quelconque des revendications 14 à 21, comprenant au moins un orifice de drainage à proximité de l'embout.

23. Cathéter selon l'une quelconque des revendications 14 à 22, comprenant des moyens pour manipuler le cathéter lors de l'insertion.

24. Cathéter selon l'une quelconque des revendications 14 à 23 comprenant en outre une pièce de raccordement.

25. Cathéter urinaire selon l'une quelconque des revendications 14 à 24, produit selon l'une quelconque des revendications 1 à 13.
